# EUROPÄISCHE PATENTSCHRIFT

(11) **EP 0 609 503 B1**
(45) Veröffentlichungstag und Bekanntmachung des Hinweises auf die Patenterteilung: **08.10.1997**
(21) Anmeldenummer: 93118331.3
(22) Anmeldetag: 12.11.1993
(51) Int. Cl.: A61B 1/32, A61B 17/28

(54) **Medizinisches Instrument**
Medical instrument
Instrument médical

(30) Priorität: 05.02.1993 DE 4303274
(43) Veröffentlichungstag der Anmeldung: 10.08.1994
(73) Patentinhaber: Richard Wolf GmbH, 75438 Knittlingen (DE)
(72) Erfinder: Heckele, Helmut, D-75438 Knittlingen (DE); Dingler, Andreas, D-75217 Birkenfeld (DE); Falk, Ernst, D-75447 Sternenfels-Diefenbach (DE)
(74) Vertreter: Vollmann, Heiko, Dipl.-Ing.

(56) Entgegenhaltungen:
- EP-A- 0 044 877
- EP-A- 0 464 463
- EP-A- 0 606 531
- WO-A-94/00061
- FR-A- 914 564
- US-A- 1 798 124

## Beschreibung

Die Erfindung betrifft ein medizinisches Instrument, insbesondere zum Manipulieren von Organen bei endoskopischen Eingriffen im Körperinneren, mit einem ausschwenkbaren Spreizkörper am distalen Instrumentenende, der mittels einer Handhabe am proximalen Instrumentenende betätigbar ist, wobei Handhabe und Spreizkörper über mindestens ein verschiebbar im Instrumentenschaft geführtes Steuerorgan miteinander verbunden sind.

Instrumente dieser Art werden insbesondere bei endoskopischen Eingriffen, beispielsweise in der Bauchhöhle eingesetzt, um Organe oder sonstige innere Körperteile zu manipulieren, in der Regel um diese in eine Stellung zu bringen und in dieser Stellung zu halten, in der sie nicht stören, insbesondere das zu untersuchende bzw. zu behandelnde Feld nicht verdecken. An derartige Instrumente sind besondere Anforderungen gestellt, da sie einerseits zum Zwecke des Einführens in die Körperhohle möglichst schlank und glatt sein sollen andererseits innerhalb der Körperhöhle einen möglichst guten Halt geben sollen. Hierzu ist es erforderlich, daß die Instrumente möglichst großflächig aufspannbar sind und in dieser Stellung auch ausreichend große Kräfte übertragen können, um beispielsweise auch ein größeres Organ sicher abhalten zu können. Weiterhin müssen die Instrumente auch in aufgespanntem Zustand so ausgebildet sein, daß eine Verletzungsgefahr weitgehend ausgeschlossen ist.

Ein gattungsgemäßes Instrument ist aus DE 40 21 153 A1 bekannt. Der dort beschriebene Organmanipulator hat sich im praktischen Einsatz gut bewährt, er erfüllt insbesondere die vorgenannten Forderungen weitgehend. Dieser Manipulator, der in unterschiedliche Stellungen aufgespannt werden kann, wird in der Regel wie ein Spatel benutzt, d.h., es können im wesentlichen nur Kräfte quer zu der von den Spreizkörpern aufgespannten Fläche sowie sehr bedingt Druckkräfte in Richtung vom proximalen zum distalen Instrumentenende aufgebracht werden. Da die Spreizkörper in aufgespanntem Zustand im wesentlichen in einer Ebene liegen, die Organe hingegen eine raumgekrümmte Oberfläche aufweisen, kann es vorkommen, daß einerseits punktuell hohe Druckbelastungen des Organs auftreten können, wenn die flächigen Spreizkörper in einem stark gekrümmten Organbereich angreifen und andererseits gelegentlich ein Abrutschen des Organs vom Manipulator nicht zu verhindern ist. Ein solches Abrutschen ist insbesondere dann, wenn gerade therapierende Eingriffe erfolgen, höchst unerwünscht und gefährlich. Des weiteren wäre es bei einigen Anwendungen wünschenswert, das Organ in die eine oder andere Richtung mit dem Manipulator drängen zu können, was mit dem vorbeschriebenen Manipulator aufgrund der Abrutschgefahr nur sehr bedingt möglich ist.

Aus DE 41 25 806 A1 ist ein optischer Gewebehaken bekannt, der aus einem Schaft mit mehreren darin axial verschiebbar angeordneten, federvorgespannten und distal zweifach abgekröpften Spreizelementen besteht, mit denen ein zu behandelndes Hohlorgan unter visueller Kontrolle fixiert werden kann. Dieses Instrument ist ausschließlich zum Fixieren eines Hohlorgans an der Bauchdecke, und zwar durch Klemmwirkung zwischen Instrumententeilen vorgesehen und geeignet. Die zuvor angesprochene seitliche Verlagerung von Organen, beispielsweise um ein Organ aus einem bestimmten Körperhöhlenbereich abzuhalten, ist mit diesem Instrument nicht möglich.

Aus DE 37 09 706 A1 ist ein weiterer Manipulator bekannt. Dieser Manipulator weist drei federelastische Drähte mit kugelförmigen Elementen an ihren distalen Enden auf. Diese Drähte sind quer zur Längsrichtung des Instruments so vorgespannt, daß sie beim Ausfahren aus einem Hüllrohr seitlich vorspringen und so ein räumliches Gebilde aufspannen. Der wesentliche Nachteil dieses Manipulators besteht darin, daß hiermit nur vergleichsweise geringe Kräfte ausgeübt werden können, da die Drähte aufgrund ihrer Federwirkung bei Belastung ausweichen. Das Instrument ist daher für den eingangs erwähnten Einsatzbereich ungeeignet.

Die Druckschrift FR-A-914 564 offenbart ein Vaginalspülgerät, das drei im Winkel von etwa 120° zu einem zentralen Spülrohr angeordnete abspreizbare bogenförmig gekrümmte Finger aufweist, die durch Betätigen eines Hebels auseinanderspreizbar sind. Die Schwenkachsen der Finger sind am proximalen Ende dieses Spülrohres angebracht. Bei diesem Instrument handelt es sich nicht um ein für endoskopische Eingriffe konzipiertes Gerät, das folglich die Erfindungsmerkmale eines Instrumentenschaftes und des darin verschiebbar geführten Steuerorgans der Spreizkörper nicht besitzt.

Ausgehend von dem einleitend beschriebenen Stand der Technik nach DE 40 21 153 A1 liegt der Erfindung die Aufgabe zugrunde, ein gattungsgemäßes Instrument so weiterzubilden, daß es unter Beibehaltung der vorbeschriebenen Eigenschaften und Einsatzmöglichkeiten auch eine sichere seitliche Führung eines Organs, also quer zur Instrumentenachse, ohne die Gefahr eines Abrutschens ermöglicht, wobei ggf. mit diesem Instrument auch noch zumindest anteilig Zugkräfte auf das Organ ausgeübt werden sollen.

Diese Aufgabe wird erfindungsgemäß durch das im Anspruch 1 definierte Instrument gelöst. Das den Spreizkörper aufweisende distale Instrumentenende ist derart abgebogen, daß in Spreizstellung eine gekrümmte Ebene zwischen Spreizkörper und dem verbleibenden Instrumentengrundkörper bzw., wenn dieses Instrumentenende ausschließlich aus Spreizkörpern besteht, zwischen den Spreizkörpern aufgespannt wird.

Durch die erfindungsgemäße Lösung in der Formgebung des distalen Instrumentenendes bzw. des daran angeordneten Spreizkörpers wird eine sichere Handhabung und Führung des zu haltenden Organs im Körperinneren gewährleistet, ohne daß hierdurch die Querschnittsabmessungen des Instrumentes in eingeschwenkter Stellung der Spreizkörper vergrößert wird. Diese besondere Formgebung, die dazu führt, daß das Instrumentenende in aufgespanntem Zustand die Form eines flachen angedeuteten Hakens oder gar eines Löffels aufweist ermöglicht es, nicht nur Querkräfte auf das Organ auszuüben, sondern dieses auch in gewisser Weise festzuhalten, so daß dieses Organ nicht nur zur Seite, sondern auch in Schrägrichtungen manipuliert werden kann. Die Erfindung sieht dabei entweder vor, daß mehrere Spreizkörper vom verbleibenden Instrumentengrundkörper abspreizbar sind.

Vorteilhafte Ausgestaltungen der Erfindung gemäß Anspruch 1 sind in den Unteransprüchen, der Beschreibung und den Figuren angegeben.

Als zweckmäßig hat es sich erwiesen, zwei Spreizkörper vorzusehen, die nahe ihren proximalen Enden am Instrumentengrundkörper bzw. am Instrumentenschaft angelenkt sind und die mit ihren freien distalen Enden abspreizbar sind, vorzugsweise zu entgegengesetzten Seiten, da dann zum einen eine gute Handhabung aufgrund der zu beiden Seiten gleichmäßigen Kraftverteilung gegeben ist und zum anderen eine vergleichsweise große Fläche zur Instrumentenachse aufgespannt wird. In diesem Falle können die Spreizkörper vorteilhaft an einer gemeinsamen Achse angelenkt sein. Soll die von den Spreizkörpern aufgespannte Ebene jedoch nicht nur in Richtung der Instrumentenachse, sondern auch quer dazu gekrümmt sein, also löffelartig, so sind zwei Achsen im Winkel zueinander anzuordnen, beispielsweise in einem spitzen Winkel von 10°.

Sowohl bei einer Ausbildung, bei der das distale Instrumentenende ausschließlich aus Spreizkörpern besteht, als auch bei einer Ausbildung, bei der in diesem Bereich ein Instrumentengrundkörper verbleibt, sind Spreizkörper und Instrumentengrundkörper in ihrer Querschnittsform vorteilhaft so aufeinander abzustimmen, daß in eingeschwenkter Stellung eine gerundete Querschnittsaußenkontur entsteht, um ein möglichst problemloses Einführen in die Körperhöhle zu ermöglichen.

In der praktischen Handhabung hat es sich als vorteilhaft erwiesen, wenn das Instrument vom proximalen zum distalen Ende hin gesehen vor den Spreizkörpern im Schaft gelenkig ausgebildet ist, um eine retrograde Abwinklung des distalen Instrumentenendes zusammen mit den daran befindlichen Spreizkörpern zu ermöglichen. Es kann so eine Schwenkbewegung des Instrumentes vom proximalen Ende, also dem Bereich der Handhabe her erfolgen, ohne daß die Stellung des Instrumentes verändert werden muß. Die Gelenkachse sollte dabei vorzugsweise quer zur Schwenkachse der Spreizkörper angeordnet sein. Wenn die Spreizkörper um unterschiedliche Achsen schwenkbar sind, sollte die Gelenkachse vorzugsweise quer zur mittleren Schwenkachse aller Spreizkörper angeordnet sein. Es können auch zwei Gelenke mit um 90° versetzten Schwenkachsen oder ein Kugelgelenk vorgesehen sein.

Für die Steuerung der Schwenkstellung des Gelenkes ist am proximalen Instrumentenende eine ensprechende Handhabe vorzusehen, die zur besseren Handhabung mit der Handhabe zur Steuerung der Spreizstellung kombiniert werden kann. Diese Schwenkstellung kann mittels einer geeigneten Handhabe feinfühlig eingestellt werden. Durch die bei entsprechender Gewindesteigung dann auftretende Selbsthemmung wird zugleich eine Arretierung in der jeweiligen Schwenkstellung erreicht, so daß ohne weitere Vorkehrungen das Instrument in dieser Winkelstellung fixiert bleibt.

Von Vorteil ist es, wenn das erfindungsgemäße Instrument mit mindestens einem Kanal versehen ist, der am proximalseitigen Instrumentenende über einen Anschluß zur Aufnahme einer Sonde oder einer Optik bzw. zum Spülen oder Absaugen von Körpersekreten oder dgl. verfügt. So ist es möglich, mit diesem Instrument nicht nur ein Organ zu manipulieren, sondern ggf. auch Stellen der Körperhöhle oder das Endoskop freizuspülen bzw. am Boden der Körperhöhle abzusaugen oder weitere therapeutische bzw. diagnostische Schritte zu unternehmen, ohne dafür das endoskopische Instrument einsetzen zu müssen. Konstruktiv kann dies dadurch erfolgen, daß innerhalb des eigentlichen Instrumentenschaftes, also im Bereich zwischen dem proximalen Anschluß und den Spreizkörpern ein Rohr angeordnet ist. Sofern im Bereich der Spreizkörper ein verbleibender Instrumentengrundkörper vorgesehen ist, kann dieses Rohr bis zum distalen Instrumentenende geführt werden. Eine bevorzugte Ausführung liegt jedoch darin, im Bereich der Spreizkörper das Rohr in einen Kanal übergehen zu lassen, wobei dieser Kanal durch die Innenseiten des Instrumentengrundkörpers und/oder der Spreizkörper begrenzt wird. Hierdurch kann dann nämlich ein variabler Querschnitt gebildet werden. Wenn das Instrument geschlossen, d.h. die Spreizkörper in eingeklappter Stellung sind, mündet der Kanal am distalen Ende.

Wie schon erwähnt, sollte die Außenkontur des Instrumentes, insbesondere im Bereich des distalen Endes gerundet ausgebildet sein. Die zueinander weisenden Seiten der Spreizkörper hingegen werden zweckmäßigerweise als Planseiten ausgebildet, so daß diese in eingeklapptem Zustand quasi wie einstückig aneinanderliegen. Durch eine solche Ausbildung wird insbesondere dann, wenn der Grundkörper einen sich zwischen die Spreizkörper erstreckenden Versteifungssteg aufweist, in eingeklapptem Zustand eine sehr hohe Stabilität des Instrumentes erreicht. Es versteht sich, daß die oberen und unteren Planflächen in Richtung der Längsachse des Instruments gekrümmt sind.

Wie bereits einleitend erwähnt, können die Schwenkachsen der Spreizkörper im Winkel zueinander angeordnet sein, wenn eine in zwei Richtungen gekrümmte Ebene aufgespannt werden soll. In der Regel wird dies jedoch nicht erforderlich sein, so daß die konstruktiv einfachere Lösung eingesetzt wird, bei der zwei oder mehrere Spreizkörper an parallelen Schwenkachsen angeordnet sind, die ihrerseits im Instrumentengrundkörper sitzen. Dabei sind die Spreizkörper in proximaler Richtung zur Bildung von Hebelarmen über die Achse hinaus verlängert und dort an ihren Enden gelenkig mit einer durch den Instrumentenschaft geführten Steuerstange verbunden, die proximalseitig in geeigneter Weise mit einer Handhabe verbunden ist. Zweckmäßigerweise ist diese Steuerstange mit einer am Griff des Instrumentes rastbar verschiebbaren Handhabe verbunden, so daß eine einfache und sichere Handhabung der Spreizfunktion gewährleistet ist und zudem eine Arretierfunktion.

Vorteilhaft ist der langgestreckte distale Instrumententeil über eine Kupplung lösbar mit dem die Handhabe aufweisenden proximalen Instrumententeil verbunden. Hierdurch ist es möglich, je nach Anforderungen den distalen Instrumententeil auszutauschen, beispielsweise. gegen einen Instrumententeil mit geradlinig verlaufenden Spreizkörpern. Hierdurch ergeben sich insbesondere kostenmäßige Vorteile, da das konstruktiv und fertigungstechnisch aufwendige, die gesamten Steuerfunktionen des Instrumentes beinhaltende Griffteil für mehrere unterschiedliche distale Instrumententeile einsetzbar ist.

Die Erfindung ist nachfolgend anhand eines in der Zeichnung dargestellten Ausführungsbeispiels näher erläutert. Es zeigen:
- Figur 1: eine Seitenansicht eines erfindungsgemäßen Instrumentes in vereinfachter Darstellung,
- Figur 2: in stark vergrößerter und teilweiser Schnittdarstellung einen Teil des distalen Instrumentenendes in Seitenansicht,
- Figur 3: eine teilweise im Schnitt dargestellte vergrößerte Seitenansicht des distalen Instrumententeiles,
- Figur 4a: einen Schnitt längs der Schnittlinie IV-IV in Figur 3,
- Figur 4b: einen Schnitt entsprechend Figur 4a einer anderen Ausführung des Instrumentes,
- Figur 4c: einen Schnitt entsprechend Figur 4a einer weiteren Ausführung des Instrumentes,
- Figur 5: eine Draufsicht auf das distale Instrumentenende nach Figur 3,
- Figur 6: einen Längsschnitt durch den proximalen Instrumententeil,
- Figur 7: einen Schnitt längs der Schnittlinie VII-VII in Figur 6 (dargestellt ohne Handhabe) und
- Figur 8: einen Längsschnitt durch die Kupplung zwischen dem distalen und dem proximalen Instrumententeil .

Das anhand der Figuren dargestellte Instrument 1 besteht im wesentlichen aus drei funktionellen Teilen, nämlich dem distalen Instrumententeil 1a, dem proximalen Instrumententeil 1b und dem diese beiden Teile verbindenden Instrumentenschaft 1c.

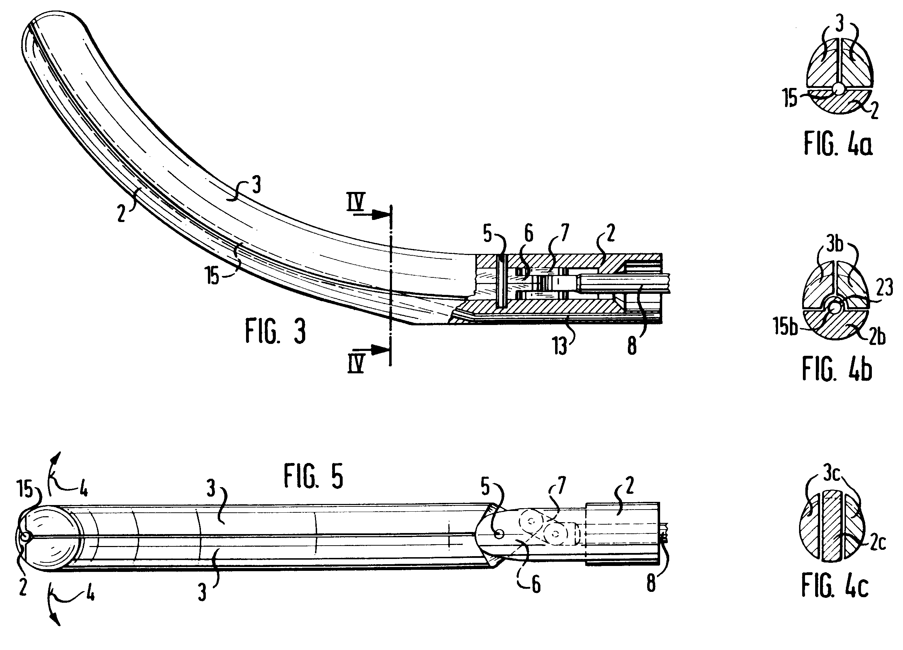

Der distale Instrumententeil 1a besteht aus einem den rohrförmigen Schaftteil 1c zum distalen Ende hin verlängernden Instrumentengrundkörper 2, der etwa halbkreisförmigen Querschnitt (Figur 4a) aufweist und aus Vollmaterial besteht. Während die Unterseite des Instrumentengrundkörpers 2 gerundet ausgebildet ist, ist seine Oberseite als plane Fläche ausgebildet, die jedoch in Achsrichtung des Instrumentenschaftes gekrümmt ist (siehe Figuren 3 und 5).

An die plane Oberseite des Instrumentengrundkörpers 2 schließen sich in der in den Figuren dargestellten geschlossenen Stellung die entsprechend plane, jedoch in Gegenrichtung gekrümmten Unterseiten von zwei Spreizkörpern 3 an. Die Spreizkörper 3 bestehen ebenfalls aus Vollmaterial und liegen in geschlossener Stellung des Instrumentes mit den zueinanderweisenden Planflächen ebenfalls bündig aneinander an, so daß sie zusammen mit dem Instrumentengrundkörper 2 eine etwa runde Querschnittskontur bilden, wie dies in Figur 4a dargestellt ist.

Die Spreizkörper 3 und der Instrumentengrundkörper 2 sind in Seitenansicht (siehe Figur 3) bogenförmig gekrümmt. Die Spreizkörper 3 sind in Richtung der Pfeile 4 (siehe Figur 5) seitlich ausschwenkbar, und zwar um eine parallele Gelenkachse 5, die innerhalb des Instrumentengrundkörpers 2 befestigt ist und diesen mit den Spreizkörpern 3 verbindet.

Die Spreizkörper 3 sind über die Gelenkachse 5 hinaus in Richtung zum proximalen Instrumentenende 1b verlängert, so daß sich Hebelarme 6 bilden, mit denen die Spreizkörper über Zwischenhebel 7 gelenkig am distalen Ende einer ersten Steuerstange 8 angelenkt sind. Bei Bewegung der Steuerstange 8 in distaler Richtung schwenken die Spreizkörper 3 in Richtung der Pfeile 4 aus.

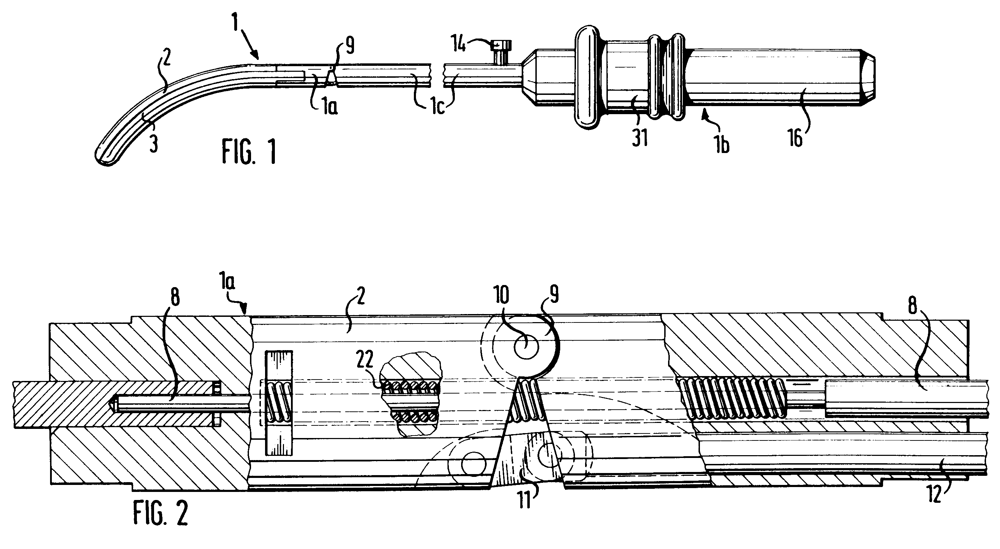

In Richtung vom proximalen zum distalen Instrumentenende gesehen ist kurz vor dem distalen Anlenkpunkt der ersten Steuerstange 8 ein Gelenk 9 vorgesehen, um das der gesamte distale Instrumententeil 1a schwenkbar ist. Die Ausbildung dieses Gelenkes 9 sowie die Durchführung der ersten Steuerstange 8 in diesem Bereich ist der Figur 2 im einzelnen zu entnehmen. Das Gelenk 9 sitzt außermittig des Schaftes 1c, seine Gelenkachse 10 ist quer zu den Gelenkachsen 5 angeordnet, so daß der gesamte distale Instrumententeil 1a retrograd um diese Gelenkachse 10 ablenkbar ist, und zwar bei dieser Ausführung nur in eine Richtung. Die Durchführung der ersten Steuerstange 8 im Bereich dieses Gelenkes ist nach Art eines Bowdenzuges gestaltet, die Steuerstange 8 ist in diesem Bereich elastisch biegbar und wird durch einen schraubenlinienförmig gewickelten Stützdraht 22 umgeben. Das Gelenk 9 ist nahe der Außenseite des Schaftes 1c angeordnet. Zu der gegenüberliegenden Seite ist der Schaft 1c vom Gelenk 9 ausgehend etwa V-förmig ausgenommen, wodurch der notwendige Freiraum zum Abbiegen geschaffen wird. Über einen Zwischenhebel 11 ist der distale Instrumententeil 1a gelenkig mit einer zweiten Steuerstange 12 derart verbunden, daß bei Bewegung der Steuerstange 12 in Richtung auf das proximale Ende der distale Instrumententeil 1a um die Gelenkachse 10 abgebogen wird und bei Bewegung der Steuerstange 12 in Gegenrichtung wieder gerade gestellt wird.

Durch den Schaft 1c sind nicht nur die beiden Steuerstangen 8 und 12, sondern auch ein Rohr 13 geführt, das Teil eines Saug-Spülkanals ist und proximalseitig in einem seitlich aus dem Schaft 1c herausgeführten Leitungsanschluß 14 endet. Distalseitig mündet dieses Rohr 13 im Bereich der Spreizkörper 3 in einem Kanal 15, der in geschlossener Stellung. des Instrumentes durch die Innenseiten der Spreizkörper 3 sowie die des Instrumentengrundkörpers 2 gebildet wird (siehe Figur 4a). Dieser Kanal 15 mündet am distalen Instrumentenende, wie aus Figur 5 ersichtlich ist. Wenn die Spreizkörper in aufgespannter Stellung sind, existiert dieser Kanal 15 nicht, der Saug-Spülkanal mündet dann am distalen Ende des Rohres 13.

Anhand der Figuren 4b und 4c sind zwei alternative Ausbildungen des distalen Instrumententeiles verdeutlicht. Bei der Ausführung nach Figur 4b ist der Kanal 15b nicht durch die Innenseiten der Spreizkörper 3b begrenzt, sondern innerhalb des Instrumentengrundkörpers 2b bis zur distalen Instrumentenspitze geführt, so daß das distale Ende dieses Kanales 15b unabhängig von der Stellung der Spreizkörper 3b ist. Der Instrumentengrundkörper ist bei dieser Ausführung etwa T-förmig ausgebildet, so daß ein den Instrumentenkanal 15b aufnehmender Versteifungssteg 23 gebildet ist, an den die Spreizkörper 3b (bei geschlossener Stellung des Instrumentes) bündig anliegen. Dieser Versteifungssteg 23 kann im Querschnitt gesehen genau so stark wie die Spreizkörper sein, so daß der Steg 23 (bei Darstellung wie in Figur 4b) das Instrument auch nach oben hin begrenzen kann. Ein solcher Versteifungssteg sorgt für zusätzliche Stabilität.

Bei der Ausführung nach Figur 4c ist der Instrumentengrundkörper 2c im Bereich der Spreizkörper als schmaler Streifen ausgebildet, der zwischen den Innenseiten der Spreizkörper liegt und gemäß Darstellung nach Figur 4c den Instrumentenquerschnitt in diesem Bereich lediglich nach oben und unten begrenzt.

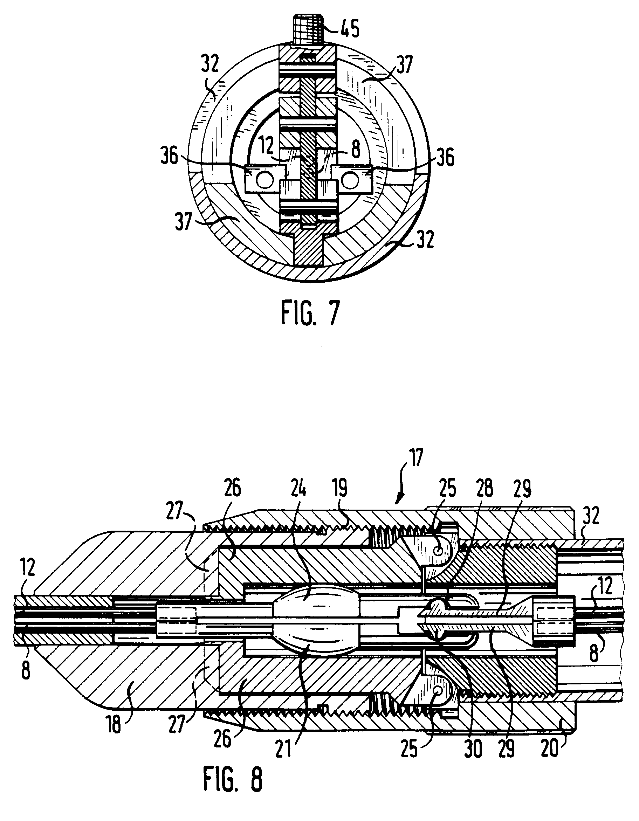

Während der Leitungsanschluß 14 vor dem am proximalen Instrumentenende angeordneten Griffteil 16 seitlich aus dem Schaft 1c herausgeführt ist, sind die Steuerstangen 8 und 12 bis in den Griffteil hineingeführt. Im distalen Bereich des Griffteiles 16 ist zunächst, wie anhand von Figur 8 dargestellt, eine Kupplung 17 vorgesehen, durch die der lnstrumentenschaft 1c mit dem daran befindlichen distalen Instrumententeil 1a lösbar mit dem proximalen Instrumententeil 1b verbunden ist. Der Instrumentenschaft 1c geht proximalseitig in einen topfförmigen Instrumententeil 18 über, der an seinem Außenumfang im proximalen Endbereich ein Außengewinde 19 aufweist, das mittels einer am Griffteil 16 festgelegten Überwurfrändelmutter 20 am Griffteil 16 befestigt ist.

In dem topfförmigen Instrumententeil 18 ist der Instrumentenschaft 1c festgelegt. Die Steuerstangen 8 und 12 weisen jeweils einen innerhalb dieses Bauteiles liegenden Kupplungsteil auf, der in Figur 8 dargestellt und mit 21 gekennzeichnet ist. Jeder Kupplungsteil 21 weist einen im Durchmesser vergrößerten Teil 24 auf. Diese Teile 24 sind innerhalb von zwei um Gelenkachsen 25 am Griffteil 16 schwenkbar gelagerte Kulissenhälften mit geringem Spiel geführt. Diese Kulissenhälften 26 wiederum werden in der montierten Stellung gemäß Figur 8 durch den topfförmigen Instrumententeil gegen ein radiales Ausschwenken formschlüssig gesichert. Um sicherzustellen, daß nach dem Verbinden der Kupplungsteile die Zuordnung zwischen Steuerelementen am Griffteil 16 und der Stellung des distalen Instrumententeiles 1a stets gleich ist, sind innerhalb des Instrumententeiles 18 zwei Ausnehmungen 27 vorgesehen, in welche die distalen Enden der Kulissenhälften 26 eingreifen. Wenn diese Enden in den Ausnehmungen 27 liegen, dann ist die definierte Instrumentenstellung erreicht und der distale Instrumententeil fest mit dem Griffteil verbunden.

Am proximalseitigen Ende jedes Kupplungsteiles 21 ist eine Aufnahme 28 etwa in Form einer längsgeteilten Hülse mit darin liegender Halbringnut vorgesehen. In jede Hälfte dieser Aufnahme 28 greift in Achsrichtung formschlüssig ein stangenförmiges Kupplungsteil 29 ein, das an seinem distalen Ende einen Halbringwulst 30 aufweist, der in der vorerwähnten Halbringnut formschlüssig liegt und somit die axiale Kraftübertragung zur Stange 8 bzw. zur Stange 12 gewährleistet. Die Aufnahme 28 kann, solange die Kupplung 17 noch nicht verriegelt ist, d. h. solange die Kulissenhälften 26 noch ausschwenken können, beim Einschieben des Kupplungsteiles 29 aufgeweitet werden, bis der Ringwulst 30 in seiner bestimmungsgemäßen Lage liegt. Die Ankupplung der zweiten Steuerstange 12 geschieht in analoger Weise.

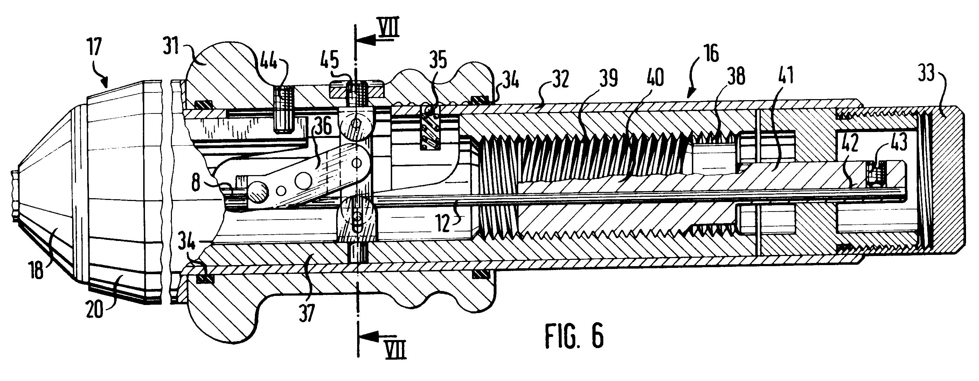

In Figur 6 ist das distale, die Kupplung 17 aufweisende Ende des Griffteils 16 verkürzt dargestellt, da diese Figur ausschließlich zur Erläuterung der mechanischen Verbindung der Handhabe 31 zu den Stangen 8 und 12 dient. Das Griffteil 16 weist einen zylindrischen hülsenartigen Grundkörper 32 auf, der proximal durch eine Abschlußkappe 33 und distal durch die Teile 18 und 20 der Kupplung 17 abgeschlossen wird; Auf diesem Grundkörper 32 ist die ringförmig ausgebildete Handhabe 31 in Axialrichtung verschiebbar sowie drehbar gelagert. Die axiale Verschiebbarkeit der Handhabe 31 dient zum Aufspreizen bzw. Schließen der Spreizkörper 3, also zum axialen Verschieben der Steuerstange 8. Beim Drehen der Handhabe 31 gegenüber dem Grundkörper 32 wird die Schwenkstellung des distalen Instrumententeils 1a geändert, d.h. die Steuerstange 12 axial bewegt. Da die anhand von Figur 7 dargestellte Ausbildung des Handhabenteils 16 unabhängig davon ist, ob das Instrument mit oder ohne Kupplung (wie in Figur 8 dargestellt) aufgebaut ist, sind die Steuerstangen 8 und 12 durchgängig mit diesem Bezugszeichen bezeichnet.

Die ringförmig ausgebildete Handhabe 31 ist über zwei Dichtungen 34 jeweils an den Enden gegenüber dem Grundkörper 32 abgedichtet. Zwischen der Handhabe 31 und dem als Gehäuse ausgebildeten Grundkörper 32 ist eine Rastvorrichtung 35 vorgesehen, so daß die Handhabe 31 in der jeweils gewählten Verschiebestellung verharrt. Im Bereich der Handhabe 31 weist der Grundkörper 16 eine etwa sich über 180° erstreckende Ausnehmung auf, in die zwei fest mit der Handhabe 31 verbundene Zapfen 44, 45 eingreifen, von denen der Zapfen 45 über ein Hebelgestänge mit dem proximalen Ende der Steuerstange 8 verbunden ist. Dieses proximale Ende ist drehbar am distalen Ende des gekröpften Hebels 36 gelagert. Das anhand der Figuren 6 und 7 im einzelnen dargestellte Hebelgestänge sorgt dafür, daß unabhängig von der Drehstellung der Handhabe 31 die axiale Verschiebestellung auf die Steuerstange 8 übertragen wird.

Innerhalb des Grundkörpers 32 sitzt eine zylindrische Hülse 37, in dem das zuvor beschriebene Hebelgestänge geführt ist. Diese Hülse 37 ist drehbar innerhalb des Grundkörpers 16 gelagert. Durch die Zapfen 44, 45 wird die jeweilige Drehstellung der Handhabe 31 auf diese Hülse 37 übertragen. Im proximalen Bereich jenseits der Handhabe 31 weist diese, innerhalb des Grundkörpers 16 axial nicht verschiebbare Hülse 37 ein Innengewinde 38 auf, das mit dem Aussengewinde 39 eines innerhalb der Hülse 37 axial verschiebbar gelagerten Kolbens 40 in Verbindung steht. Dieser Kolben weist in Richtung zum proximalen Ende eine Verlängerung 41 auf, die in dem von der Kappe 33 abgedeckten Teil endet und somit nach Entfernen der Kappe 33 zugänglich ist. Der Kolben 40 und die Verlängerung 41 sind von einer Längsbohrung 42 durchsetzt, in der die Steuerstange 12 mittels einer Schraube 43 (siehe Figur 6) festgelegt ist. Diese Schraube dient zum Festlegen der Grundeinstellung.

Die Handhabe 31 ist aus der in Figur 7 dargestellten mittleren Stellung gegenüber dem Gehäuse 16 um etwa 90° nach links oder nach rechts drehbar. Dadurch wird die innerhalb des Grundkörpers 16 gelagerte Hülse 37 gedreht, wodurch der Kolben 40, der drehfest gelagert ist, axial verschoben wird. Auf diese Weise wird auch die Steuerstange 12 axial verschoben und somit die Schwenkstellung des Gelenkes 9 geändert. Mit der Handhabe 31 ist also eine zentrale Bedienung des Instrumentes mit nur einer Hand möglich.

Wie bereits angedeutet, sind zahlreiche Detailvarianten des Instrumentes möglich, so kann es wahlweise mit oder ohne Kupplung aufgebaut werden. Auch kann, sofern dies gewünscht wird, auf den feststehenden Instrumentengrundkörper 2 im Bereich der Spreizkörper 3 ggf. vollständig verzichtet werden. Es können auch je nach Bedarf ein oder mehrere Spreizkörper vorgesehen werden. Auch kann, sofern dies gewünscht wird, eine elektromotorische Verstellung der Steuerstangen 8 und 12 erfolgen. Dann befinden sich anstelle der Handhabe 31 entsprechende Schalter am Griffteil 16.

## Patentansprüche

1. Medizinisches Instrument zum Manipulieren von Organen bei endoskopischen Eingriffen im Körperinneren, mit folgenden Merkmalen:
- das Instrument weist einen proximalen Instrumententeil (1b) und einen distalen Instrumententeil (1a) auf, die über einen Instrumentenschaft (1c) miteinander verbunden sind, wobei der distale Instrumententeil (1a) aus einem den Instrumentenschaft (1c) zum distalen Ende verlängernden Instrumentengrundkörper (2) besteht,
- am distalen Instrumententeil (1a) sind mindestens zwei starre, zu entgegengesetzten Seiten ausschwenkbare Spreizkörper (3) angelenkt,
- am proximalen Instrumententeil (1b) ist eine Handhabe (31) vorgesehen, mit der die Spreizstellung der Spreizkörper (3) steuerbar ist,
- Handhabe (31) und Spreizkörper (3) sind über ein im Instrumentenschaft (1c) verschiebbar geführtes Steuerorgan (8) verbunden,
- in ungespreizter Stellung liegen die Spreizkörper (3) bündig an dem Instrumentengrundkörper (2),
- Spreizkörper (3) and Instrumentengrundkörper (2) sind zu einer Seite der Ausrichtung des Instrumentenschaftes (1c) derart bogenförmig gekrümmt ausgebildet, daß die Spreizkörper (3) in Spreizstellung zusammen mit dem Instrumentengrundkörper (2) eine gekrümmte Fläche aufspannen,
- die Spreizkörper (3) weisen freie distale Enden auf, mit denen sie gegenüber dem Instrumentengrundkörper (2) um eine gemeinsame Schwenkachse (5) oder um parallele Achsen ausschwenkbar sind.

2. Medizinisches Instrument nach Anspruch 1, dadurch gekennzeichnet, daß das distale Instrumententeil (1a) bei eingeschwenkter Stellung der Spreizkörper (3) eine runde Querschnittsaußenkontur aufweist.

3. Medizinisches Instrument nach Anspruch 1 oder 2, dadurch gekennzeichnet, daß das distale Instrumententeil (1a) ein zur Schwenkachse (5) der Spreizkörper (3) proximal angeordnetes Gelenk (9) zum Schwenken des gesamten distalen Instrumenteils (1a) aufweist, dessen Gelenkachse (10) vorzugsweise quer zur Schwenkachse (5) der Spreizkörper (3) angeordnet ist.

4. Medizinisches Instrument nach Anspruch 3, dadurch gekennzeichnet, daß die Schwenkstellung des Gelenkes (9) über die Handhabe (31) am proximalen Instrumententeil (1b) steuerbar und festlegbar ist.

5. Medizinisches Instrument nach einem der vorhergehenden Ansprüche, dadurch gekennzeichnet, daß innerhalb des Instrumentes (1) mindestens ein Kanalvolumen mit proximalseitigem Anschluß (14) vorgesehen ist, der durch ein längs innerhalb des Instrumentes verlaufendes Rohr (13) gebildet ist.

6. Medizinisches Instrument nach einem der vorhergehenden Ansprüche, dadurch gekennzeichnet, daß das Rohr (13) vor oder im Bereich der Spreizkörper (3) endet und dort in einen Kanal (15) übergeht, der innerhalb des Instrumentengrundkörpers (2) liegt und/oder durch zueinander weisende Seiten der Spreizkörper (3) begrenzt ist.

7. Medizinisches Instrument nach Anspruch 5 oder 6, dadurch gekennzeichnet, daß das Kanalvolumen am distalen Instrumentenende endet.

8. Medizinisches Instrument nach einem der vorhergehenden Ansprüche, dadurch gekennzeichnet, daß die Spreizkörper (3) in eingeschwenkter Stellung an ihren zueinanderweisenden Planseiten aneinanderstoßen und an ihren planen Unterseiten von der planen Oberseite des Instrumentengrundkörpers (2) abgestützt sind.

9. Medizinisches instrument nach einem der vorhergehenden Ansprüche dadurch gekennzeichnet, daß Teile der Spreizkörper in proximaler Richtung zur Bildung eines Hebelarms (6) über die Schwenkachse (5) hinausragen und dort an ihren Enden an einer durch den Instrumentenschaft (1c) geführten gemeinsamen Steuerstange (8) angelenkt sind.

10. Medizinisches Instrument nach einem der vorhergehenden Ansprüche, dadurch gekennzeichnet, daß die Steuerstange (8) bis in einen als Griff (16) ausgebildeten Instrumententeil verläuft und dort mit der in Achsrichtung am Griff (16) rastbar verschiebbaren Handhabe (31) verbunden ist.

11. Medizinisches Instrument nach einem der vorhergehenden Ansprüche, dadurch gekennzeichnet, daß der Instrumentengrundkörper (2b) (Fig. 4b) einen sich bis zwischen die Spreizkörper (3b) (Fig. 4b) erstreckenden Versteifungssteg (23) aufweist, der in geschlossenem Zustand des Instrumentes an den Innenseiten der Spreizkörper (3b) anliegt.

12. Medizinisches Instrument nach einem der vorhergehenden Ansprüche, dadurch gekennzeichnet, daß der langgestreckte distale Instrumententeil (1a) über eine Kupplung (17) lösbar mit dem die Handhabe (31) aufweisenden proximalen Instrumententeil (1b) verbunden ist.

## Claims

1. A medical instrument for manipulating organs during endoscopic operations in the inside of the body, with the following features:
- the instrument comprises a proximal instrument part (1b) and a distal instrument part (1a) which are connected to one another via an instrument shank (1c), wherein the distal instrument part (1a) comprises an instrument base body (2) elongating the instrument shank (1c) to the distal end,
- at the distal instrument part (1a) there are linked at least two rigid splaying bodies (3) which can be pivoted out on opposing sides,
- at the proximal instrument part (1b) there is provided a handle (31) with which the splayed position of the splaying bodies (3) can be controlled,
- the handle (31) and splaying bodies (3) are connected via a control member (8) displaceably guided in the instrument shank (1c),
- in the unsplayed position the splaying bodies (3) lie flush with the instrument base body (2),
- the splaying bodies (3) and instrument base body (2), to one side of the alignment of the instrument shank (1c), are formed curved in an arched shape such that the splaying bodies (3) in the splayed position together with the instrument base body (2) stenter a curved surface,
- the splaying bodies (3) comprise free distal ends with which they may be pivoted out, with respect to the instrument base body (2), about a common pivoting axis (5) or about parallel axes.

2. A medical instrument according to claim 1, characterised in that the distal instrument part (1a) with a pivoted-in position of the splaying bodies (3), comprises a round cross-sectional outer contour.

3. A medical instrument according to claim 1 or 2, characterised in that the distal instrument part (1a) comprises a link (9), arranged proximally to the pivoting axis (5) of the splaying bodies (3), for pivoting the whole distal instrument part (1a), and whose link axis (10) is preferably arranged transversely to the pivoting axis (5) of the splaying bodies (3).

4. A medical instrument according to claim 3, characterised in that the pivot position of the link (9) can be controlled and fixed via the handle (31) at the proximal instrument part (1b).

5. A medical instrument according to one of the preceding claims, characterised in that within the instrument (1) there is provided at least one lumen volume with a connection (14) on the proximal side, said lumen volume being formed by a tube (13) running longitudinally through the instrument.

6. A medical instrument according to one of the preceding claims, characterised in that the tube (13) ends before or in the region of the splaying bodies (3) and here blends into a lumen (15) which lies within the instrument base body (2) and/or is limited by sides, facing one another, of the splaying bodies (3).

7. A medical instrument according to claim 5 or 6, characterised in that the lumen volume ends at the distal instrument end.

8. A medical instrument according to one of the preceding claims, characterised in that the splaying bodies (3) in the pivoted-in position about one another at their flat sides facing one another and at their flat lower sides are supported by the flat upper side of the instrument base body (2).

9. A medical instrument according to one of the preceding claims, characterised in that parts of the splaying bodies project beyond the pivoting axis (5) in the proximal direction for forming a lever arm (6) and here are linked at their ends to a common control rod (8) guided through the instrument shank (1c).

10. A medical instrument according to one of the preceding claims, characterised in that the control rod (8) runs up to an instrument part formed as a grip (16) and here is connected to the handle (31) which is lockably displaceable on the grip (16) in the axial direction.

11. A medical instrument according to one of the preceding claims, characterised in that the instrument base body (2b) (Fig. 4b) comprises a reinforcing web (23) extending up to between the splaying bodies (3b) (Fig. 4b), the reinforcing web resting on the inner sides of the splaying bodies (3b) in the closed condition of the instrument.

12. A medical instrument according to one of the preceding claims, characterised in that the longitudinally extended distal instrument part (1a) is releasably attached to the proximal instrument part (1b) comprising the handle (31) via a coupling (17).

## Revendications

1. Instrument médical pour manipuler des organes lors d'interventions endoscopiques à l'intérieur du corps, présentant les caractéristiques suivantes:
- l'instrument comporte une partie proximale (1b) et une partie distale (1a), qui sont reliées l'une à l'autre par l'intermédiaire d'une tige tubulaire (1c), la partie distale (1a) de l'instrument étant constituée d'un corps de base (2) de l'instrument, prolongeant la tige tubulaire (1c) de l'instrument en direction de l'extrémité distale,
- sur la partie distale (1a) de l'instrument, sont articulés au moins deux corps écarteurs rigides (3) aptes à pivoter vers des côtés opposés,
- sur la partie proximale (1b) de l'instrument, il est prévu une poignée (31), à l'aide de laquelle la position d'écartement des corps écarteurs (3) peut être commandée,
- la poignée (31) et les corps écarteurs (3) sont reliés par l'intermédiaire d'un organe de commande (8) guidé en translation dans la tige tubulaire (1c) de l'instrument,
- dans la position non écartée, les corps écarteurs (3) sont placés au ras du corps de base (2) de l'instrument,
- les corps écarteurs (3) et le corps de base (2) de l'instrument sont réalisés sous une forme incurvée en arc vers un côté de la direction d'orientation de la tige tubulaire (1c) de l'instrument, ceci d'une façon telle que les corps écarteurs (3), dans la position d'écartement, tendent, conjointement avec le corps de base (2) de l'instrument, une surface incurvée,
- les corps écarteurs (3) présentent des extrémités distales libres, par lesquelles ils peuvent pivoter vers l'extérieur, par rapport au corps de base (2) de l'instrument, autour d'un axe de pivotement commun (5) ou autour d'axes parallèles.

2. Instrument médical selon la revendication 1, caractérisé en ce que la partie distale (1a) de l'instrument présente, dans la position rentrée par pivotement des corps écarteurs (3), une section transversale de contour extérieur circulaire.

3. Instrument médical selon la revendication 1 ou 2, caractérisé en ce que la partie distale (1a) de l'instrument comporte une articulation (9) pour le pivotement de l'ensemble de la partie distale (1a) de l'instrument, qui est disposée du côté proximal par rapport à l'axe de pivotement (5) des corps écarteurs (3) et dont l'axe (10) est disposé de préférence transversalement à l'axe de pivotement (5) des corps écarteurs (3).

4. Instrument médical selon la revendication 3, caractérisé en ce que la position de pivotement de l'articulation (9) peut être commandée et immobilisée par l'intermédiaire de la poignée (31) située sur la partie proximale (1b) de l'instrument.

5. Instrument médical selon l'une des revendications précédentes, caractérisé en ce qu'à l'intérieur de l'instrument (1), il est prévu au moins un volume formant canal qui est muni d'un raccord (14) côté proximal et qui est défini par un tube (13) s'étendant longitudinalement à l'intérieur de l'instrument.

6. Instrument médical selon l'une des revendications précédentes, caractérisé en ce que le tube (13) se termine en amont de ou dans la région des corps écarteurs (3) et rejoint en cet endroit un canal (15), qui est situé à l'intérieur du corps de base (2) de l'instrument et/ou est délimité par des faces, tournées l'une vers l'autre, des corps écarteurs (3).

7. Instrument médical selon la revendication 5 ou 6, caractérisé en ce que le volume formant canal se termine à l'extrémité distale de l'instrument.

8. Instrument médical selon l'une des revendications précédentes, caractérisé en ce que les corps écarteurs (3), dans la position rentrée par pivotement, sont jointifs au niveau de leurs faces planes tournées l'une vers l'autre et sont soutenus, au niveau de leurs faces inférieures planes, par la face supérieure plane du corps de base (2) de l'instrument.

9. Instrument médical selon l'une des revendications précédentes, caractérisé en ce que des parties des corps écarteurs, en vue de la formation d'un bras de levier (6), font saillie en direction proximale au-delà de l'axe de pivotement (5) et sont articulées en cet endroit, à leurs extrémités, à une tige de commande commune (8) guidée à travers la tige tubulaire (1c) de l'instrument.

10. Instrument médical selon l'une des revendications précédentes, caractérisé en ce que la tige de commande (8) s'étend jusque dans une partie de l'instrument réalisée sous forme d'un manche (16) et y est reliée à la poignée (31) déplaçable en direction axiale sur le manche (16), tout en pouvant être arrêtée par encliquetage.

11. Instrument médical selon l'une des revendications précédentes, caractérisé en ce que le corps de base (2b) de l'instrument (figure 4b) présente une nervure de rigidification (23) qui s'étend jusque dans la région située entre les corps écarteurs (3b) (figure 4b) et qui, dans l'état fermé de l'instrument, s'applique sur les faces intérieures des corps écarteurs (3b).

12. Instrument médical selon l'une des revendications précédentes, caractérisé en ce que la partie distale (1a), étendue en longueur, de l'instrument est reliée d'une manière détachable, par l'intermédiaire d'un accouplement (17), à la partie proximale (1b) de l'instrument, qui comporte la poignée (31).
